# EUROPEAN PATENT SPECIFICATION

(11) **EP 1 616 577 B1**
(45) Date of publication and mention of the grant of the patent: **21.09.2011**
(21) Application number: 04728020.1
(22) Date of filing: 16.04.2004
(51) Int. Cl.: A61P 25/00, A61K 31/517, A61P 27/00, A61K 38/43, A61K 31/00

(54) **2-chloro-6,7-dimethoxy-N-5 [5-(1)H- indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride for use in repairing corneal perception.**
2-Chloro-6,7-dimethoxy-N-5 [5-(1)H- indazolyl]quinazolin-4-amin, N-(1-Benzyl-4-piperidinyl)-1H-indazol-5-amin dihydrochlorid, 4-[2-(2,3,4,5,6-pentafluorophenyl)acryloyl]zimtsäure und Fasudil-Hydrochlorid zur Verwendung bei der Wiederherstellung der Hornhautwahrnehmung.
2-chloro-6,7-diméthoxy-N-5 [5-(1)H- indazolyl]quinazoline-4-amine, N-(1-benzyl-4-pipéridinyl)-1H-indazole-5-amine dihydrochloride, acide 4-[2-(2,3,4,5,6-pentafluorophenyl)acryloyl]cinnamic et fasudil hydrochloride pour son utilisation dans la réparation de la perception cornéen.

(30) Priority: 18.04.2003 JP 2003114819; 11.07.2003 JP 2003273177
(43) Date of publication of application: 18.01.2006
(73) Proprietor: Senju Pharmaceutical Co., Ltd., Osaka-shi, Osaka 541-0046 (JP)
(72) Inventor: TAKAYAMA, Yoshiko, Kobe-shi, Hyogo 6550003 (JP); NAKAMURA, Yoshikuni, Hyogo 6540053 (JP); INOUE, Jun, Kobe-shi, Hyogo 6540103 (JP); AZUMA, Mitsuyoshi, Nishinomiya-shi, Hyogo 6620031 (JP)
(74) Representative: von Kreisler Selting Werner
(86) International application number: PCT/JP2004/005456
(87) International publication number: WO 2004/091662

(56) References cited:
- WO-A1-01/68607
- WO-A1-02/083175
- WO-A1-02/100833
- WO-A2-02/076977
- JP-A- 2000 044 513
- JP-A- 2003 073 357
- US-A1- 2002 119 140
- NEGISHI M. ET AL: 'Regulation of neurite formation by Rho family GTPases' SEIKAGAKU, J. OF JAPANESE BIOCHEMICAL SOCIETY vol. 74, no. 5, May 2002, pages 395 - 398, XP002980610
- NIKOLIC M.: 'The role of Rho GTPases and associated kinases in regulating neurite outgrowth' INT'L J. BIOCHEM CELL BIOL. vol. 34, no. 7, July 2002, pages 731 - 745, XP002980611
- LEHMANN M ET AL: 'Inactivation of Rho Signaling Pathway Promotes CNS Axon Regeneration' J NEUROSCI vol. 19, no. 17, 01 September 1999, pages 7537 - 7547, XP002952684
- DERGHAM P ET AL: 'Rho signaling pathway targeted to promote spinal cord repair' J NEUROSCI vol. 22, no. 15, 01 August 2002, pages 6570 - 6577, XP002980612

## Description

### Technical Art

The present invention relates to an agent for promoting corneal neuritogenesis comprising a Rho protein inhibitor, and an agent for the recovery or improvement of corneal sensitivity or the treatment of dry eye, based on the promotion of corneal neuritogenesis.

Since corneal nerve is severed by corneal surgeries such as Laser photorefractive keratectomy (PRK), Laser-Assisted-In-Situ Keratomileusis (LASIK), keratoplasty and the like, the corneal sensitivity is said to decrease generally for about 3 weeks to one year. For example, it has been reported that the corneal nerve is apparently severed after LASIK (Tuuli U. Linna et al., Experimental Eye Research 66: 755-763, 1998), and the corneal sensitivity decreases in a corneal region where, after LASIK, neurogram is not observed or the nerve bundle is too short to create connection (Tuuli U. Linna et al., Investigative Ophthalmology & Visual Sciences, 41: 393-397, 2000).

It has been demonstrated that the corneal hyposensitivity after PRK and LASIK causes lower lacrimal gland response and decreased lacrimal fluid (Ang, Robert T. et al., Current Opinion in Ophthalmology 12: 318-322, 2001). As a result of the functional decrease of corneal sensitivity, patients after a corneal surgery blink less number of times, problematically showing the symptoms of dry eye. In the patients with dry eye, lacrimal hypofunction gives rise to corneal hyposensitivity, which, upon combination with further lacrimal hypofunction, problematically aggravates the condition of corneal surface.

At present, however, recovery of corneal sensitivity after a corneal surgery is left to spontaneous recovery, and in the treatment of dry eye, no active treatment is provided to recover corneal sensitivity.

In addition, corneal hyposensitivity is caused by the diseases accompanying corneal neurodegeneration, such as neuroparalytic keratopathy, corneal ulcer, diabetic keratopathy and the like.

Rho protein is a low molecular weight G protein included in the Rho family (containing Rho, Rac, Cdc42, etc.), and is known to be involved in actin cytoskeleton organization and neurite retraction reaction.

For example, C3 enzyme, a Rho protein inhibitor, is known to extend cell protrusion of 3T3 fibroblast (Hirose, M. et al., The Journal of Cell Biology, 141: 1625-1636, 1998), and a method of promoting the growth of central nerve axon by the administration of an effective amount of Rho protein inhibitor to patients is disclosed (JP-T-2001-515018 and EP-1,011,330-A). In addition, a Rho kinase inhibitor, which is among the effector molecules of Rho protein, is known to have an axon extension action of retinal ganglion cells, and exhibit a regeneration promoting action on the optic nerve cell (WO 02/83175 and EP-1,142,585-A). WO 03/020281 teaches that a compound capable of promoting nerve regeneration or neurite extension can be used for the treatment of a djsease state caused by a corneal nerve disorder after surgery such as LASIK and the like. As examples of such compound, neotrofin, which is a neurotrophic factor stimulating substance, and the like are shown, but no description is found nor suggestion as to an Rho protein inhibitor.

As to the trigeminal nerve, it has been reported that, in a rat trigeminal nerve tissue culture (trigeminal tract in whole mount cultures) system, extension of neurotrophin-induced nerve axon of nerve growth factor (NGF) and the like is inhibited by a Rho activator (lysophosphatidic acid), and facilitated by introduction of dominant negative Rho into a cell (Ozdinler, P. Hande et al., The Journal of Comparative Neurology, 438:377-387, 2001). Meanwhile, there is a description that whether Rho is effective for trigeminal tract nerve axon extension in the absence of neurotrophin is unknown, and the effect of a Rho protein inhibitor on the trigeminal nerve has not been elucidated.

On the other hand, it is disclosed that, since a compound having a Rho activating effect has a corneal epithelial migrating action and C3 enzyme, which is a Rho protein inhibitor, inhibits migration of the corneal epithelium thereof, a compound having a Rho activating effect is useful for corneal failure such as corneal ulcer, corneal epithelial abrasion, keratitis and the like (JP2000-264847A and EP-1,142,585A).

### Disclosure of the Invention

The present invention provides a pharmaceutical agent that shows functional recovery of corneal sensitivity in patients having functional decrease of corneal sensitivity, from patients after corneal surgery such as Laser photorefractive keratectomy (PRK), Laser-In-Situ Keratomileusis (LASIK), keratoplasty and the like.

The present inventors have studied for the purpose of providing a new type of pharmaceutical agent that recovers corneal sensitivity after corneal surgery or improves the condition of corneal sensitivity in a dry eye and first found that Rho protein inhibitor has a neuritogenesis-promoting effect for the trigeminal nerve (hereinafter sometimes to be referred to as corneal nerve) cell. They have further studied based on these findings, and completed the present invention that utilizes a Rho protein inhibitor as a drug for the recovery of corneal sensitivity and the like.

Accordingly, the present invention relates to
1. A pharmaceutical composition for promoting the extension of the corneal nerve axon, which composition comprises a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride.
2. A pharmaceutical composition for the recovery of corneal sensitivity, which composition comprises a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride.
3. A pharmaceutical composition for the treatment of dry eye, which composition comprises a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride.
4. Use of a a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride for the production of a pharmaceutical composition for promoting corneal neuritogenesis.
5. Use of a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride for the production of a pharmaceutical composition for promoting the extension of the corneal nerve axon.
6. Use of a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentaffuorophenyl)acryloyl]cinnamic acid and fasudil hydrochloride for the production of a pharmaceutical composition for the recovery of corneal sensitivity.
7. Use of a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride for the production of a pharmaceutical composition for the treatment of dry eye.

### Brief Description of the Drawings

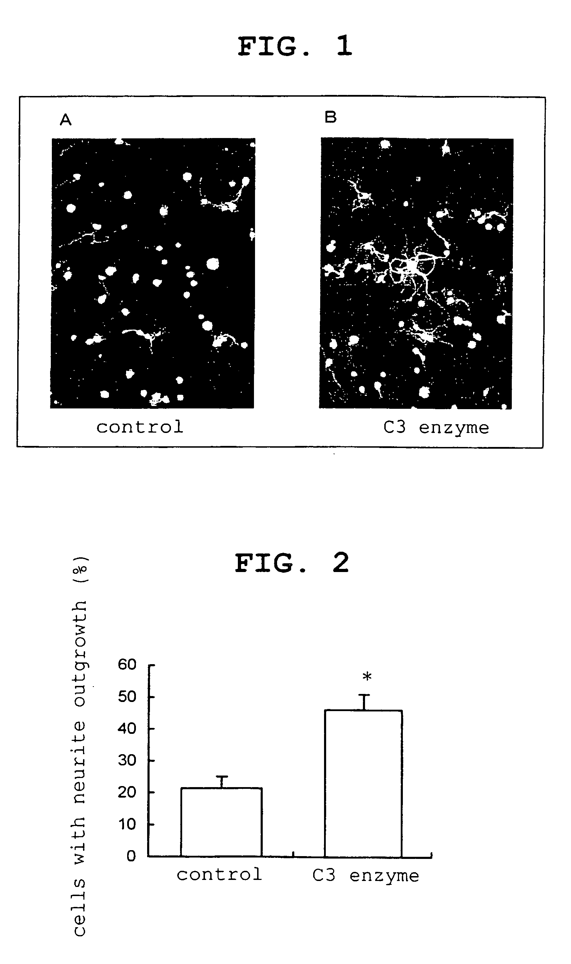

Fig. 1 is a fluorescence microscopic image of cultured rabbit trigeminal nerve cells in Experimental Example 1, wherein A is rabbit trigeminal nerve cells cultured for 24 hr in a C3 enzyme non-addition culture medium and B shows cells cultured for 24 hrs in a culture medium containing C3 enzyme at a final concentration of 2 µg/mL.
Fig. 2 shows the rate (%) of neuritogenetic cell to the total cell number in Experimental Example 1, wherein the vertical axis shows a percentage of neuritogenetic cells to the total cells, each value shows mean±standard error of 3 experiments and * shows a significant difference (p<0.05) relative to the control.

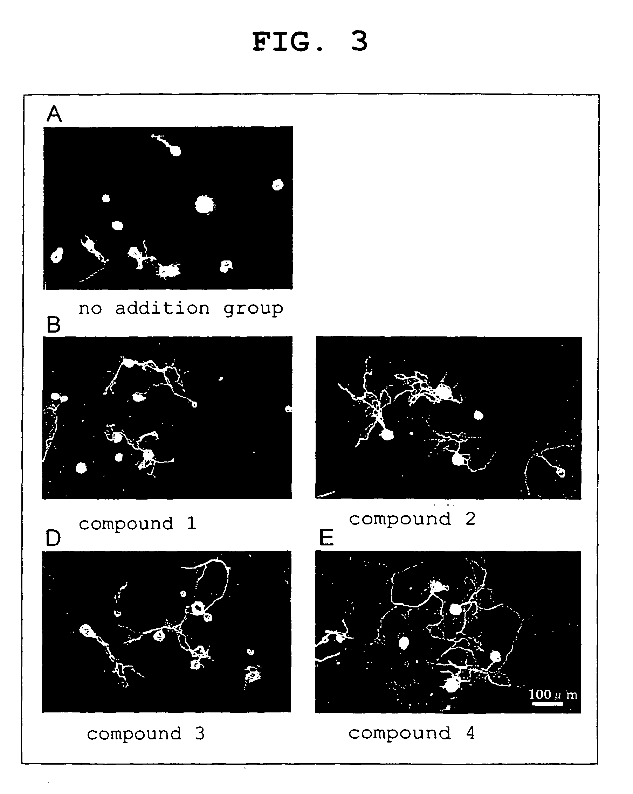

Fig. 3 is a fluorescence microscopic image of cultured rabbit trigeminal nerve cells in Experimental Example 2, wherein A shows the cells cultured in a test substance non-addition culture medium, B shows the cells cultured in a compound 1 addition (final concentration 10 µM) culture medium, C shows the cells cultured in a compound 2 addition (final concentration 10 µM) culture medium, D shows the cells cultured in a compound 3 addition (final concentration 1 µM) culture medium, and E shows the cells cultured in a compound 4 addition (final concentration 10 µM) culture medium for 48 hrs.

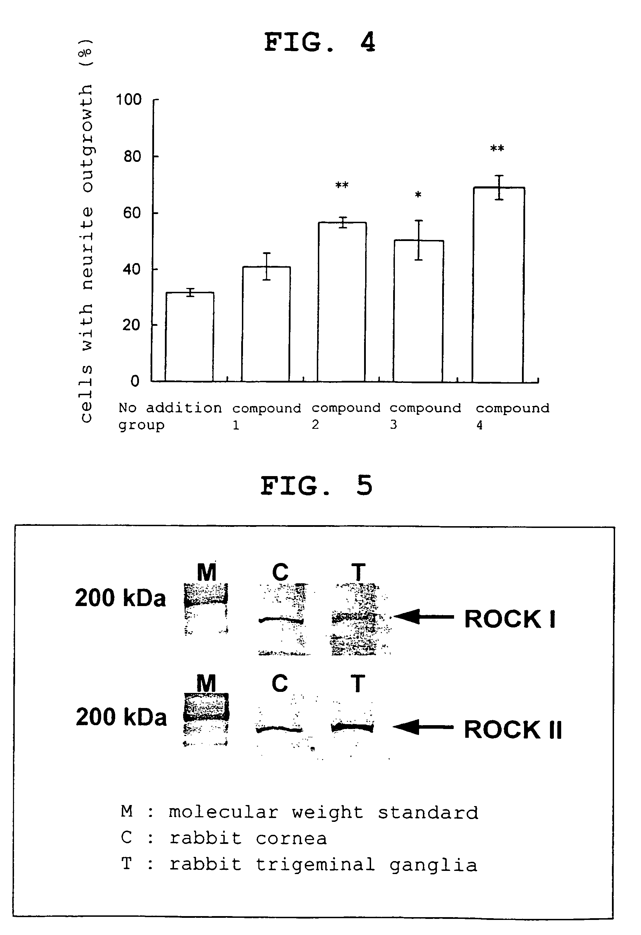

Fig. 4 is a graph showing the rate (%) of neuritogenetic cell to the counted total cell number in Experimental Example 2, wherein each value shows mean±standard error of 3 experiments, * shows a significant difference (p<0.05) relative to the non-addition group, and ** shows a significant difference (p<0.01) relative to the non-addition group.
Fig. 5 shows the results of ROCK I and ROCK II Western blotting of rabbit cornea (C) and trigeminal ganglia (T) in Experimental Example 3.

### Detailed description of the Invention

In the present specification, the "Rho protein inhibitor" refers to the compounds selected from 2-chloro-6, 7-dimethoxy-N-[5-(1)-indazolyl]quinazoline-4-amine, N-(1-benzyl-4-piperidinyl)-1H-indozole-5-amine dihydrochloride, 4-[2-(2, 3, 4, 5, 6-pentafluorophenyl)acryloyl]cinnamic acid and fasudil hydrochloride that inhibit an inactive GDP-binding Rho protein from being activated into an active GTP-binding Rho protein, an antibody against Rho protein or Rho protein fragment, and the like, and any inhibitor that inhibits the action of an effector molecule that transduces the action of Rho protein, such as Rho kinase (ROCK), and the like. The "corneal nerve" refers to annular plexus formed in the surrounding cornea under the control of trigeminal nerve, which is a sensory neuron, stroma plexus distributed reticulately in corneal stroma, sub-epithelial plexus formed immediately below Bowman's membrane, and basal cell plexus and nerve fiber formed immediately after penetrating Bowman's membrane. In the present invention, "neurite" refers to a protrusion (dendrite and axon) from the cell body of neuron (nerve cell), and "genesis" refers to an outgrowth and/or extension of the aforementioned neurite from the cell body. It is clear to those of ordinary skill in the art what level of neuritogenesis is regarded as promotion. The promotion of neuritogenesis can be confirmed by, for example, fluorescent staining the nerve cell and observing the cell condition with a fluorescence microscope. In addition, the observation results using a fluorescence microscope may be analyzed using an image analysis software and the like. Moreover, the state of neuritogenesis can be numerically expressed by statistically processing the results. As a still another method, a substance constituting nerve cell body and neurite, such as neurofilament, may be labeled with an antibody that recognizes the same and a horseraddish peroxidase (HRP)-conjugated antibody reactive with said antibody, HRP is allowed to develop color and the amount of the neurofilament is determined by measuring the absorbance and used as an index of the neuritogenesis.

As the Rho protein inhibitor, for example, Exoenzyme C3 (sometimes simply referred to as C3 enzyme in the present specification), Toxin A, Toxin B and Rho kinase inhibitor can be mentioned but it is not part of the invention

The Rho kinase inhibitor (hereinafter sometimes to be referred to as ROCK inhibitor) is the compound described in JP61-227581A (USP 4,678,783) i.e., FASUDIL hydrochloride; the compound described in WO 00/57914 and JP2000-44513A i.e., 4-[2-(2,3,4,5,6-pentafluorophenyl)acryloyl]cinnamic acid the compound described in WO 02/076977 (EP-1,370,552-A and 1,370,553-A) i.e.) 2-chloro-6,7-dimethoxy-N-[5-1H-indazolyl]quinazoline-4-amine ; and the compound described in WO 02/100833 i.e) N-(1-benzyl-4-piperidinyl)-1H-indazole-5-amine dihydrochloride monohydrate are mentioned.

In the following explanation, pharmaceutical agents and compositions containing the Rho protein inhibitor to be used in the present invention are also sometimes referred to collectively as "a pharmaceutical agent of the present invention".

The pharmaceutical agent of the present invention is useful for the recovery from functional decrease in the corneal sensitivity of mammals (e.g., human, rat, mouse, rabbit, bovine, pig, dog, cat and the like), wherein the corneal nerve is damaged or cut or defective. For example, it is useful as a therapeutic drug for recovering decreased corneal sensitivity after PRK, LASIK and the like, decreased corneal sensitivity accompanying corneal neurodegeneration, such as neuroparalytic keratopathy, corneal ulcer, diabetic keratopathy and the like, or as a therapeutic drug for dry eye having decreased corneal sensitivity.

The pharmaceutical agent of the present invention is systemically or topically administered. Systemically, it is orally administered, and parenterally, it is administered as intravenous injection, subcutaneous injection, intramuscular injection and the like. Topically, it is administered to the eye.

As the dosage form of the pharmaceutical agent of the present invention, solid agents such as powder, granule, tablet, capsule, suppository and the like; liquids such as syrup, injection, eye drop and the like; and the like can be mentioned.

For the production of the pharmaceutical agents of the present invention as granules and tablets, any dosage form can be produced by using, for example, excipients (lactose, sucrose, glucose, starch, microcrystalline cellulose and the like), lubricants (magnesium stearate, talc, stearic acid, calcium stearate and the like), disintegrants (starch, carmellose sodium, calcium carbonate and the like), binders (starch paste solution, hydroxypropylcellulose solution, carmellose solution, gum arabic solution, gelatin solution, sodium alginate solution and the like) and the like. For granules and tablets, a coating film may be formed using suitable coating agents (gelatin, sucrose, gum arabic, carnauba wax and the like), enteric coatings (e.g., cellulose acetate phthalate, metacrylic acid copolymer, hydroxypropylcellulose phthalate, carboxymethylethylcellulose and the like) and the like.

For the production of the pharmaceutical agents as capsule, a mixture of suitable excipients such as magnesium stearate, calcium stearate, talc, light silicic anhydride and the like for improving flowability and glidability, microcrystalline cellulose, lactose and the like for flowability under pressurization, as well as the above-mentioned disintegrant and the like added as appropriate is uniformly admixed or granulated or granulated, coated with a suitable coating agent to form a film and packed in a capsule, or encapsulation-molded with a capsule base having increased plasticity, which contains a suitable capsule base (gelatin and the like), glycerin or sorbitol and the like. These capsules may contain coloring agents, preservatives [sulfur dioxide, parabens (methyl paraoxybenzoate, ethyl paraoxybenzoate or propyl paraoxybenzoate)] and the like as necessary. The capsule may be a conventional one, an enteric coated capsule, a gastric coated capsule or a release control capsule. When an enteric capsule is produced, a compound coated with an enteric coated agent or the above-mentioned suitable excipients are added to a compound and packed in a conventional capsule or a capsule itself may be coated with an enteric coating agent, or an enteric polymer may be used as a base for molding.

For the production of the pharmaceutical agent of the present invention as a suppository, a base for suppository (e.g., cacao butter, macrogol and the like) can be appropriately selected and used.

For the production of the pharmaceutical agents as syrup, for example, stabilizers (sodium edetate and the like), suspending agents (gum arabic, carmellose and the like), corrigents (simple syrup, glucose and the like), aromatic and the like can be appropriately selected and used.

For the production of the pharmaceutical agent of the present invention as an injection or eye drop, it can be produced by dissolving or dispersing the inhibitor in a solution appropriately containing pharmaceutically acceptable additives such as isotonicity agents (sodium chloride, potassium chloride, glycerin, mannitol, sorbitol, boric acid, borax, glucose, propylene glycol and the like), buffers (phosphate buffer, acetate buffer, borate buffer, carbonate buffer, citrate buffer, Tris buffer, glutamate buffer, ε-aminocaproate buffer and the like), preservatives (p-oxybenzoates, chlorobutanol, benzyl alcohol, benzalkonium chloride, sodium dehydroacetate, sodium edetate, boric acid, borax and the like), thickeners (hydroxyethyl cellulose, hydroxypropyl cellulose, polyvinyl alcohol, polyethylene glycol and the like), stabilizers (sodium bisulfite, sodium thiosulfate, sodium edetate, sodium citrate, ascorbic acid, dibutylhydroxytoluene and the like), pH adjusting agents (hydrochloric acid, sodium hydroxide, phosphoric acid, acetic acid and the like), and the like.

While the amount of the additives to be used for the above-mentioned syrup, injection and eye drop varies depending on the kind of the additives to be used, use and the like, they may be added at a concentration capable of achieving the purpose of the additive, and an isotonicity agent is generally added in 0.5 - 5.0 w/v% to make the osmotic pressure 229 - 343 mOsm. In addition, a buffer is added in 0.01 - 2.0 w/v%, a thickener is added in 0.01 - 1.0 w/v%, and a stabilizer is added in 0.001 - 1.0 w/v%. A pH adjusting agent is appropriately added to generally achieve a pH of - 3 - 9, preferably 4 - 8.

For particular use of the pharmaceutical agent of the present invention as an eye drop, the lower limit of the concentration of the Rho protein inhibitor contained in the pharmaceutical agents of the present invention is adjusted to generally 0.00001 w/v%, preferably is 0.00005 w/v% or more preferably is 0.0001 w/v% and the upper limit is adjusted to 0.1 w/v%, preferably is 0.05 w/v%, more preferably is 0.01 w/v%, further preferably is 0.005 w/v% or more further preferably is 0.001 w/v%.

While the dose of the pharmaceutical agent of the present invention varies depending on the target disease, symptom, subject of administration, a kind of Rho protein inhibitor, administration method and the like, when, for example, it is topically administered to the eye of an adult after PRK surgery as an agent for the recovery of corneal sensitivity, for example, a liquid eye drop containing 0.001 w/v% of C3 enzyme or 0.003 w/v% of Rho protein inhibitor such as N-(1-benzy1-4-piperizinyl)-1H-indazole-5-amine-2hydrochloride. 1/2 hydrate is preferably instilled into the eye several times a day at 20 to 50 µL per dose.

In addition, when the pharmaceutical agent of the present invention is orally administered to an adult as a corneal sensitivity recovery agent after LASIK surgery, for example, a tablet containing about 10 mg of a Rho protein inhibitor such as 4-[2-(2,3,4,5,6-pentafluorophenyl)acryloyl]cinnamic acid is preferably administered once or twice a day.

### Examples

The present invention is explained in more detail by referring to the following Experimental Examples and Examples, which are not to be construed as limitative.

### Experimental Example 1

Promoting effect on neuritogenesis in cultured rabbit trigeminal nerve cells

### 1) Animals used

Japanese White Rabbits (2-3 days old) purchased from Fukusaki Rabbit Warren were used.

### 2) Test substance

C3 enzyme [manufactured by Upstate; Exoenzyme C3 (recombinant enzyme expressed in E. coli); Catalog #13-118, Lot #23330]

### 3) Test method

Cell culture: The trigeminal nerve cell was isolated according to the report of Chan et al. (Chan, Kuan Y. and Haschke, Richard H., Exp. Eye Res., 41: 687-699, 1985). To be specific, under ether anesthesia, after cardiac perfusion of rabbit with saline, the trigeminal ganglia was removed, dispersed using a nerve dispersion solution (SUMITOMO BAKELITE Co., Ltd.), and the cells were inoculated in a 8-well culture slide (BECTON DICKINSON Co., Ltd.) coated with polylysine. The number of cells was about 3 × 10³ cells per well and the culture conditions were 5% CO₂, 95% air and humidity 100% at 37°C. For cell culture, Neurobasal medium (GIBGO) added with B27 supplement (GIBCO; 0.02 mL/mL culture solution) and L-glutamic acid (GIBCO; final concentration 1 mM) were used, and C3 enzyme (2 µg/mL final concentration) was added to the medium immediately after cell inoculation and the cells were cultured for 24 hr.

Immunostaining: After 24 hr of culture, the cells were fixed with 4% paraformaldehyde at room temperature for 2 hr, and nerve cell body and neurite were fluorescence stained using an anti-neurofilament 200 antibody (manufactured by Sigma) that specifically recognizes neurofilaments which are intermediate filaments specific to a nerve cell and a fluorescent secondary antibody (manufactured by Molecular Probes) reactive therewith. The stained cells were imported as images (one image: 1.83 mmx1.36 mm) from the fluorescence microscope into a computer, and the whole cell number (t) of each image was counted. Along therewith, the length of cell neurite was measured using an image analysis software (MacSCOPE, manufactured by MITANI CO.), and the cells having a neurite with a length of not less than twice the diameter of the cell body were counted as neuritogenetic cell (a). Multiple images were imported until the total of the whole cells in respective images (t₁+t₂+...+tₙ=Σt) reached about not less than 100. Then the rate (%) of the total neuritogenetic cell number (a₁+a₂+...+aₙ=Σa) to the total cell number (Σt) was calculated. To compare C3 enzyme addition group and non-addition group (control group) with regard to this rate, t-test was performed and a critical rate of less than 5% was taken as significant.

### 4) Test results

Fig. 1 shows fluorescence microscopic images of cultured rabbit trigeminal nerve cells, wherein A shows cells of the control group cultured for 24 hrs in a C3 enzyme non-addition culture medium and B shows cells cultured for 24 hrs in a culture medium containing C3 enzyme at a final concentration of 2 µg/mL, and Fig. 2 shows the rate of the number of neuritogenetic cells to the total number of cells of each group.

The rate of the neuritogenetic cell was about 21% of the total cell number in the control group, and about 46% of the total cell number in the C3 enzyme addition group. Addition of C3 enzyme significantly increased the number of cells showing neurite outgrowth (Fig. 2).

From the foregoing, it has been found that C3 enzyme having a Rho inhibitor activity promotes neurite outgrowth of trigeminal nerve cells.

### Experimental Example 2

Neurite outgrowth promoting effect in cultured rabbit trigeminal nerve cells

### 1) Animals used

Japanese White Rabbits (2-3 days old) purchased from KITAYAMA LABES Co., Ltd. were used.

### 2) Test substance

As a ROCK inhibitor, 2-chloro-6,7-dimethoxy-N-[5-1H-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride were used.

2-Chloro-6,7-dimethoxy-N-[5-1H-indazolyl]quinazoline-4-amine (hereinafter to be indicated as compound 1) used was synthesized according to Reference Example 1. N-(1-benzyl-4-piperidinyl)-1H-indazole-5-amine dihydrochloride·1/2 hydrate (hereinafter to be indicated as compound 2) used was synthesized according to Reference Example 2. 4-[2-(2,3,4,5,6-Pentafluorophenyl)acryloyl]cinnamic acid (hereinafter to be indicated as compound 3) used was synthesized according to Reference Example 3. Fasudil hydrochloride (hereinafter to be indicated as compound 4) used was a commercially available fasudil hydrochloride hydrate injection, "Eril Injection 30 mg" (manufactured by Asahi Kasei Corporation).

### 3) Cell culture

Rabbit trigeminal nerve cells were isolated in the same manner as in Experimental Example 1. For cell culture, a culture medium obtained by adding B27 Supplement (manufactured by GIBCO; final concentration 2% v/v) and L-glutamine (manufactured by GIBCO; concentration 1 mM) to neurobasal culture medium (manufactured by GIBCO) was used. A circular cover glass (diameter 12 mm; manufactured by SUMITOMO BAKELITE) after polylysine/laminin coating was placed in each well of a 24 well plate, and cells were plated on the cover glass at about 3 × 10³ cells/well. After cell adhesion to the cover glass (about 2 hrs), the above-mentioned culture medium was changed to a culture medium containing each test substance (compound 1, final concentration 10 µM; compound 2, final concentration 10 µM; compound 3, final concentration 1 µM; compound 4, final concentration 10 µM) and the cells were cultured for 48 hrs. The culture conditions were 5% CO₂, 95% air, humidity 100%, 37°C.

### 4) Immunostaining

The cells after culture for 48 hrs were fixed for 2 hrs at room temperature using 4% paraformaldehyde.

A specimen fixed using an anti-neurofilament 200 antibody (manufactured by Sigma) that recognize neurofilaments, which are intermediate filaments specific to nerve cell, and a fluorescent secondary antibody (manufactured by Molecular Probes) reactive therewith was fluorescence stained and the stained cells were detected using a fluorescence microscope. The stained images were imported into a computer at 1 image: 1.83 mmx1.36 mm. The whole cell number (t) of each image was counted. Along therewith, the length of cell neurite and the diameter of cell body were measured using an image analysis software (MacSCOPE, manufactured by MITANI CO.), and the cells having a neurite with a length of not less than twice the diameter of the cell body were counted as neuritogenetic cell (a). Then the rate (%) of the total neuritogenetic cell number (a₁+a₂+...+aₙ=Σa) to the total cell number (Σt) was calculated.

### 5) Statistical processing

The non-addition group (control) and the test substance addition group were compared for the rate of neuritogenetic cells by the Dunnet's multiple test, and a critical rate of less than 5% was taken as significant.

### 6) Test results

Fig. 3 shows fluorescence microscopic images of cultured rabbit trigeminal nerve cells.

Fig. 3A shows the cells cultured for 48 hrs in a test substance non-addition culture medium, Fig. 3B shows the cells cultured for 48 hrs in a compound 1 addition culture medium, Fig. 3C shows the cells cultured for 48 hrs in a compound 2 addition culture medium, Fig. 3D shows the cells cultured for 48 hrs in a compound 3 addition culture medium, and Fig. 3E shows the cells cultured for 48 hrs in a compound 4 addition culture medium.

Fig. 4 shows the rate of the numbers of neuritogenetic cells of non-addition group and respective test substance addition groups relative to the total number of cells. The rate of the neuritogenetic cells relative to the total cells was about 31% for the non-addition group, about 41% for the compound 1 addition group, about 57% for the compound 2 addition group, about 51% for the compound 3 addition group, and about 70% for the compound 4 addition group, and the test substance addition groups showed a significant increase or a tendency toward increase in the rate of the neuritogenetic cells.

From the above results, it has been clarified that a ROCK inhibitor shows a neuritogenesis-promoting effect on trigeminal nerve cells.

### Reference Example 1

### Synthesis of 2-chloro-6,7-dimethoxy-N-[5-1H-indazolyl]quinazoline-4-amine (WO 02/076977, Example 1)

2,4-Dichloro-6,7-dimethoxyquinazoline (8.6 g, 64.58 mmol), 5-aminoindazole (4.8 g, 36.04 mmol) and potassium acetate (7.351 g, 74.91 mmol) were added to tetrahydrofuran/purified water (138 mL/62 mL), and the mixture was stirred overnight at room temperature. Purified water (130 mL) was added to the mixture to allow crystal precipitation. The precipitated crystals were washed with purified water and recrystallized from DMF-H₂O to give the object 2-chloro-6,7-dimethoxy-N-[5-1H-indazolyl]quinazoline-4-amine as a slight yellow powder.
mp 278.7-283.8°C.
¹H-NMR (300 MHz, DMSO-d₆) δ 3.93 (s, 3H), 3.96 (s, 3H), 7.16 (s, 1H), 7.60 (m, 2H), 7.90 (s, 1H), 8.03 (s, 1H), 8.12 (s, 1H), 9.94 (s, 1H), 13.13 (br s, 1H).
Anal. Calcd. for C₁₇H₁₅N₅O₂Cl·½H₂O: C, 55.97, H, 4.14, N, 19.20. Found: C, 56.05, H, 4.46, N, 19.22.

### Reference Example 2

### Synthesis of N-(1-benzyl-4-piperidinyl)-1H-indazole-5-amine dihydrochloride 1/2 hydrate (WO 02/100833, Example 1)

To a solution of 1-benzyl-4-piperidone (14.21 g, 75.1 mmol, 13.92 mL) in 1,2-dichloroethane (80 mL) were added 5-aminoindazole(10.0 g, 75.10 mmol), triacetoxysodium borohydride (11.5 g, 52.6 mmol) and acetic acid (4.29 mL, 75.1 mmol) at room temperature, and the mixture was stirred overnight at room temperature. Then, the reaction mixture was poured into aqueous 1N-sodium hydroxide solution and extracted with ethyl acetate. The organic layer was washed with saturated brine and dried over anhydrous magnesium sulfate. The solvent was evaporated under reduced pressure and the obtained residue was recrystallized from methanol to give N-(1-benzyl-4-piperidinyl)-1H-indazole-5-amine (7.8 g, 34%).
mp 150.1-152.2°C.
¹H-NMR (300 MHz, DMSO-d₆) δ 1.39 (m, 2H), 1.94 (m, 2H), 2.08 (t, 2H, J = 10.8), 2.79 (d, 2H, J = 11.4), 3.19 (m, 1H), 3.47 (s, 3H), 5.11 (d, 1H, J = 7.8), 6.68 (br s, 1H), 6.83 (dd, 1H, J = 8.9, 1.7), 7.20-7.37 (m, 6H), 12.58 (br s, 1H).
Anal. Calcd. for C₁₉H₂₂N₄: C, 74.48, H, 7.24, N, 18.29.
Found: C, 74.42, H, 7.27, N, 18.37

To a solution of the obtained N-(1-benzyl-4-piperidinyl)-1H-indazole-5-amine (6.0 g, 19.58 mmol) in tetrahydrofuran (60 mL) were added 1N-hydrochloric acid/ether solution (38 mL) and 4N-hydrochloric acid/ethyl acetate solution (13 mL) at room temperature, and the mixture was stirred at room temperature for 30min. The precipitated solid was collected by filtration and recrystallized from methanol to give N-(1-benzyl-4-piperidinyl)-1H-indazole-5-amine dihydrochloride 1/2 hydrate (4.32g, 56%).
mp 193.0-194.6°C.
¹H-NMR (300 MHz, DMSO-d₆) δ2.18 (m, 1.75H), 2.51 (m, 0.25H), 2.98 (m, 1.5H), 3.17 (m, 0.5H), 3.41 (m, 2H), 3.68 (m, 0.75H), 3.90 (m, 0.25H), 4.25 (m, 1.5H), 4.46 (m, 0.5H), 7.40-7.64 (m, 6H), 7.59 (m, 1H), 7.70 (m, 1H), 7.92 (m, 1H), 8.20 (s, 1H), 11.02 (br s, 0.75H), 11.53 (br s, 0.25H).
Anal. Calcd. for C₁₉H₂₂N₄2HCl ½H₂O: C, 58.76, H, 6.49, N, 14.43. Found: C, 58.49, H, 6.48, N, 14.45.

### Reference Example 3

### Synthesis of 4-[2-(2,3,4,5,6-pentafluorophenyl)acryloyl]cinnamic acid (JP2000-44513A,

### Example 8)

### Step 1

Under a nitrogen atmosphere, (2,3,4,5,6-pentafluorophenyl)acetic acid (20 g, 93.5 mol), ether (7 mL) and concentrated sulfuric acid (0.5 mL) were added to isobutene (27 mL) while cooling with dry ice, and the mixture was stirred in a pressure-resistant tube at room temperature for 3 days. To a mixture of 10% aqueous sodium hydrogen carbonate solution and ice was added the reaction mixture cooled with dry ice and the mixture was stirred. Ether was added and the mixture was extracted. The organic layer was washed with saturated brine, dried over anhydrous magnesium sulfate and concentrated under reduced pressure to give (2,3,4,5,6-pentafluorophenyl)acetic acid t-butyl ester (24.4 g, 97%).

### Step 2

To a solution of 4-formylbenzoic acid (20 g, 0.133 mol) in pyridine (138 mL) were added ethyl malonate monopotassium salt (46 g, 0.270 mol), p-toluenesulfonic acid monohydrate (50 g, 0.263 mol) and piperidine (2.0 mL) and the mixture was gradually heated. The mixture was stirred at 120°C for 1.5 hrs. Under ice-cooling, the reaction mixture was acidified with 2N hydrochloric acid and the precipitate was collected to give 4-carboxycinnamic acid ethyl ester (26.58 g, 91%) as crystals.

### Step 3

Under a nitrogen atmosphere, to a solution of 4-carboxycinnamic acid ethyl ester (5.0 g, 22.7 mmol) in chloroform (15 mL) was added dropwise thionyl chloride (8.4 mL), dimethylformamide (1 drop) was added and the mixture was heated under reflux for 30 min. The reaction mixture was concentrated under reduced pressure to give acid chloride.

Under a nitrogen atmosphere, to a solution of (2,3,4,5,6-pentafluorophenyl)acetic acid t-butyl ester (6.35 g, 23.5 mmol) obtained in step 1 in tetrahydrofuran (100 mL) was added dropwise a 1M toluene solution (26 mL) of lithium bis(trimethylsilyl)amide while cooling with dry ice. Five min later, a solution of acid chloride obtained in step 2 in tetrahydrofuran (100 mL) was added dropwise. At 20 min from the completion of the dropwise addition, the mixture was stirred at room temperature for 1.5 hrs. 5% Aqueous citric acid solution (120 mL) was added to the reaction mixture and the mixture was extracted with ether. The organic layer was washed with water and saturated brine, dried over anhydrous magnesium sulfate and concentrated under reduced pressure. The obtained residue was purified by silica gel column chromatography to give 4-[(2RS)-2-(t-butoxycarbonyl)-2-(2,3,4,5,6-pentafluorophenyl)acetyl]cinnamic acid ethyl ester (4.83 g, 44%) as crystals.

### Step 4

A solution of 4-[(2RS)-2-(t-butoxycarbonyl)-2-(2,3,4,5,6-pentafluorophenyl)acetyl]cinnamic acid ethyl ester (5.6 g, 11.6 mmol) obtained in Step 3 in dioxane (24 ml) was placed in a pressure resistant tube, and concentrated hydrochloric acid (24 ml) was added. The mixture was stirred for 4 hrs while heating to 130°C. The reaction mixture was ice-cooled and the precipitate was collected by filtration to give 4-[(2,3,4,5,6-pentafluorophenyl)acetyl]cinnamic acid (3.7 g, 90%) as crystals. A solution of 4-[(2,3,4,5,6-pentafluorophenyl)acetyl]cinnamic acid (2.03 g, 5.7 mmol) in dioxane (115 mL) was placed in a pressure resistant tube, paraformaldehyde (0.7 g), dimethylamine hydrochloride (1.86 g, 22.8 mmol), acetic acid (10 drops) and anhydrous magnesium sulfate (8 g) were added and the mixture was stirred overnight while heating to 130°C. Under ice-cooling, the reaction mixture was acidified with 0.1N hydrochloric acid and extracted with ethyl acetate. The organic layer was washed with saturated brine, dried over anhydrous magnesium sulfate and concentrated under reduced pressure. The precipitate was collected by filtration to give the title compound (1.46 g, 93%) as crystals.
mp 214.0-217.0°C.
¹H-NMR (300 MHz, CDCl₃) δ 6.25 (s, 1H), 6.30 (s, 1H), 6.46 (d, 1H, J = 16.2), 7.57 (d, 2H, J = 8.4), 7.64 (d, 1H, J = 15.9), 7.78 (d, 2H, J = 8.4)

### Experimental Example 3

### Protein expression of ROCK I, ROCK II in rabbit trigeminal ganglia and corneal tissue

### 1) Animals used

Male Japanese White Rabbit purchased from KITAYAMA LABES Co., Ltd. was used.

### 2) Preparation of tissue soluble protein

The animal was euthanized and trigeminal ganglia and cornea were removed. The removed tissues were placed in ice-cooled phosphate-buffered saline (manufactured by Invitrogen), washed, placed in ice-cooled 20 mM Tris-hydrochloride buffer (pH 7.5) containing 0.1% Triton X-100 (manufactured by Pharmacia Biotech) and 1 tablet/10 ml of Protease inhibitor cocktail (complete, Mini; manufactured by Roche) and ultrasonicated. A cell crude disruption solution derived from each tissue, which was obtained by ultrasonication, was centrifuged (10,000xg, 15 min, 4°), and the supernatant was recovered to give a tissue soluble protein solution. The protein amount in the solution was quantified with BCA Protein Assay Reagent (manufactured by PIERCE).

### 3) Detection of ROCK I, ROCK II using Western Blotting

ROCK I and ROCK II contained in the prepared tissue soluble protein were detected by Western Blotting. A prepared solution containing 25 µg of protein was separated by electrophoresis using 8% SDS-polyacrylamide gel (manufactured by TEFCO), and the protein separated in the gel was electrically transferred onto a PVDF membrane (Immobilon-P; Millipore). The membrane with the transferred protein was blocked with 5% skim milk, reacted with a goat anti-ROCK I antibody or a goat anti-ROCK II antibody (both manufactured by SANTA CRUZ BIOTECHNOLOGY) and secondarily labeled with alkaline phosphatase(AP) conjugated anti-goat IgG antibody (manufactured by Bio-Rad, Richmond, CA). The antigen was immunodetected using an AP coloring kit (manufactured by Bio-Rad, Richmond, CA).

### 4) Test results

The results of Western Blotting are shown in Fig. 5. It was confirmed that both ROCK I and ROCK II were expressed at a protein level in a soluble protein of rabbit trigeminal ganglia and corneal tissue.

### Example 1: tablet (not part of Invention)

| | | |
|---|---|---|
| C3 enzyme | 10 | mg |
| Lactose | 80 | mg |
| Starch | 17 | mg |
| Magnesium stearate | 3 | mg |
| Microcrystalline cellulose | 10 | mg |

Using the above components as materials for one tablet, tablets are formed according to a conventional method. The tablets may be coated as necessary with a conventional enteric coating (e.g., hydroxypropylmethylcellulose phthalate and the like), or a sugar coating or a film (e.g., ethylcellulose). The principal agent, C3 enzyme, may be changed to compound 1, 2, 3 or 4. By changing the mixing ratio of additives, tablets containing the principal agent by 20 mg, 5 mg, 1 mg, 0.5 mg or 0.1 mg/tablet can be prepared.

### Example 2: capsule (not part of the invention)

| | | |
|---|---|---|
| C3 enzyme | 50 | mg |
| Mannitol | 75 | mg |
| Starch | 17 | mg |
| Calcium stearate | 3 | mg |

Using the above components as materials for one capsule, they are uniformly mixed, granulated according to a conventional method and packed in a hard capsule. Before packing, the granules may be coated as necessary with a conventional enteric coating (e.g., hydroxypropylmethylcellulose phthalate), a sugar coating or a film (e.g., ethylcellulose). The principal agent, C3 enzyme, may be changed to compound 1, 2, 3 or 4. By changing the mixing ratio of additives, capsules containing the principal agent by 20 mg, 10 mg, 5 mg, 1 mg, 0.5 mg or 0.1 mg/capsule can be prepared.

### Example 3: injection (not part of the invention)

| | | |
|---|---|---|
| C3 enzyme | 750 mg | |
| Carboxymethylcellulose sodium | 500 mg | |
| Water for injection | total amount 100 mL | |

The above components are aseptically admixed according to a conventional method to give an injection. The principal agent, C3 enzyme, may be changed to compound 1, 2, 3 or 4. By changing the mixing ratio of additives, injections containing the principal agent by 1000 mg, 500 mg, 200 mg or 100 mg/100 mL can be prepared.

### Example 4: eye drop (not part of the invention)

| | |
|---|---|
| C3 enzyme | 5 mg |
| Boric acid | 700 mg |
| Borax | suitable amount (pH 7.0) |
| Sodium chloride | 500 mg |
| Hydroxymethylcellulose | 0.5 g |
| Sodium edetate | 0.05 mg |
| Benzalkonium chloride | 0.005 mg |
| Sterilized purified water | total amount 100 mL |

Sterilized purified water (80 mL) is heated to about 80°C, hydroxymethylcellulose is added and the mixture is stirred until the liquid temperature reaches room temperature. C3 enzyme, sodium chloride, boric acid, sodium edetate and benzalkonium chloride are added to this solution to allow dissolution. A suitable amount of borax is added to adjust its pH to 7. Sterilized purified water is added to measure up to 100 mL. The principal agent, C3 enzyme, may be changed to compound 1, 2, 3 or 4. By changing the mixing ratio of additives, eye drops containing the principal agent at 1 w/v%, 0.5 w/v%, 0.3 w/v%, 0.1 w/v%, 0.05 w/v%, 0.03 w/v%, 0.01 w/v%, 0.003 w/v% and 0.001 w/v% can be prepared.

### Example 5: eye drop (not part of the invention)

| | |
|---|---|
| C3 enzyme | 10 mg |
| D-mannitol | 4.5 g |
| Sodium dihydrogen phosphate | 0.1 g |
| Sodium hydroxide | suitable amount (pH 7.0) |
| Sterilized purified water | total amount 100 mL |

C3 enzyme, D-mannitol and sodium dihydrogen phosphate are added to sterilized purified water (80 mL) to allow dissolution. A suitable amount of sodium hydroxide is added to adjust its pH to 5.0. Sterilized purified water is added to measure up to 100 mL. The prepared eye drop is aseptically filtered with a membrane filter and filled in a disposable (unit dose) container and sealed. The principal agent, C3 enzyme, may be changed to compound 1, 2, 3 or 4. By changing the mixing ratio of additives, eye drops containing the principal agent at 1 w/v%, 0.5 w/v%, 0.3 w/v%, 0.1 w/v%, 0.05 w/v%, 0.03 w/v%, 0.005 w/v%, 0.003 w/v% and 0.001 w/v% can be prepared.

### Industrial Applicability

Since the pharmaceutical agent of the present invention, which contains a Rho protein inhibitor, has a neuritogenesis-promoting effect on trigeminal nerve cells, it is useful for improving functional decrease of corneal sensitivity associated with corneal nerve damage and the like, and the symptoms of dry eye associated with a functional decrease in the corneal sensitivity. Specifically, application of a Rho protein inhibitor is expected to provide an improving effect on decreased corneal sensitivity after cataract surgery or LASIK surgery, decreased corneal sensitivity and dry eye associated with corneal neurodegeneration such as neuroparalytic keratopathy, corneal ulcer, diabetic keratopathy and the like.

## Claims

1. A pharmaceutical composition for use in promoting the extension of the corneal nerve axon, which composition comprises a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentaffuorophenyl)acryloyl]cinnamic acid and fasudil hydrochloride.

2. A pharmaceutical composition for use in the recovery of corneal sensitivity, which composition comprises a compound selected from 2-chloro-6,7-dimethoxy-N-[5-(1)-indazolyl]quinazoline-4,amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride.

3. A pharmaceutical composition for use in the treatment of dry eye, which composition comprises a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(z,3,4,5,6-pentafluorophenyl)acryloyl]cinnamic acid and fasudil hydrochloride.

4. Use of a a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride for the production of a pharmaceutical composition for promoting corneal neuritogenesis.

5. Use of a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride for the production of a pharmaceutical composition for promoting the extension of the corneal nerve axon.

6. Use of a compound selected from 2-chloro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride for the production of a pharmaceutical composition for the recovery of corneal sensitivity.

7. Use of a compound selected from 2-chioro-6,7-dimethoxy-N-[5-(1)-indazolyl]quinazoline-4-amine, N-(1-benzyl-4-piperidinyl)-1H-indazole-5-amine dihydrochloride, 4-[2-(2,3,4,5,6-pentafluorophenyl)acryloyl]cinnamic acid and fasudil hydrochloride for the production of a pharmaceutical composition for the treatment of dry eye.

## Patentansprüche

1. Pharmazeutische Zusammensetzung zur Verwendung bei der Förderung der Verlängerung des Axons kornealer Nerven, wobei die Zusammensetzung eine Verbindung umfasst, die aus 2-Chlor-6,7-dimethoxy-N-[5-(1)-indazolyl]chinazolin-4-amin, N-(1-Benzyl-4-piperidinyl)-1H-indazol-5-amin-Dihydrochlorid, 4-[2-(2,3,4,5,6-Pentafluorphenyl)acryloyl]zimtsäure und Fasudil-Hydrochlorid ausgewählt ist.

2. Pharmazeutische Zusammensetzung zur Verwendung bei der Rückgewinnung der kornealen Empfindlichkeit, wobei die Zusammensetzung eine Verbindung umfasst, die aus 2-Chlor-6,7-dimethoxy-N-[5-(1)-indazolyl]chinazolin-4-amin, N-(1-Benzyl-4-piperidinyl)-1H-indazol-5-amin-Dihydrochlorid, 4-[2-(2,3,4,5,6-Pentafluorphenyl)acryloyl]zimtsäure und Fasudil-Hydrochlorid ausgewählt ist.

3. Pharmazeutische Zusammensetzung zur Verwendung bei der Behandlung von Keratokonjunktivitis sicca, wobei die Zusammensetzung eine Verbindung umfasst, die aus 2-Chlor-6,7-dimethoxy-N-[5-(1)-indazolyl]chinazolin-4-amin, N-(1-Benzyl-4-piperidinyl)-1H-indazol-5-amin-Dihydrochlorid, 4-[2-(2,3,4,5,6-Pentafluorphenyl)acryloyl]zimtsäure und Fasudil-Hydrochlorid ausgewählt ist.

4. Verwendung einer Verbindung, die aus 2-Chlor-6,7-dimethoxy-N-[5-(1)-indazolyl]chinazolin-4-amin, N-(1-Benzyl-4-piperidinyl)-1H-indazol-5-amin-Dihydrochlorid, 4-[2-(2,3,4,5,6-Pentafluorphenyl)acryloyl]zimtsäure und Fasudil-Hydrochlorid ausgewählt ist, zur Herstellung einer pharmazeutischen Zusammensetzung zur Förderung der kornealen Neuritogenese.

5. Verwendung einer Verbindung, die aus 2-Chlor-6,7-dimethoxy-N-[5-(1)-indazolyl]chinazolin-4-amin, N-(1-Benzyl-4-piperidinyl)-1H-indazol-5-amin-Dihydrochlorid, 4-[2-(2,3,4,5,6-Pentafluorphenyl)acryloyl]zimtsäure und Fasudil-Hydrochlorid ausgewählt ist, zur Herstellung einer pharmazeutischen Zusammensetzung zur Förderung der Verlängerung des Axons kornealer Nerven.

6. Verwendung einer Verbindung, die aus 2-Chlor-6,7-dimethoxy-N-[5-(1)-indazolyl]chinazolin-4-amin, N-(1-Benzyl-4-piperidinyl)-1H-indazol-5-amin-Dihydrochlorid, 4-[2-(2,3,4,5,6-Pentafluorphenyl)acryloyl]zimtsäure und Fasudil-Hydrochlorid ausgewählt ist, zur Herstellung einer pharmazeutischen Zusammensetzung zur Rückgewinnung der kornealen Empfindlichkeit.

7. Verwendung einer Verbindung, die aus 2-Chlor-6,7-dimethoxy-N-[5-(1)-indazolyl]chinazolin-4-amin, N-(1-Benzyl-4-piperidinyl)-1H-indazol-5-amin-Dihydrochlorid, 4-[2-(2,3,4,5,6-Pentafluorphenyl)acryloyl]zimtsäure und Fasudil-Hydrochlorid ausgewählt ist, zur Herstellung einer pharmazeutischen Zusammensetzung zur Behandlung von Keratokonjunktivitis sicca.

## Revendications

1. Composition pharmaceutique pour son utilisation pour favoriser l'extension de l'axone du nerf cornéen, ladite composition comprenant un composé choisi parmi la 2-chloro-6,7-diméthoxy-N-[5-(1)-indazolyl]quinazoline-4-amine, le dichlorhydrate de N-(1-benzyl-4-pipéridinyl)-1H-indazole-5-amine, l'acide 4-[2-(2,3,4,5,6-pentafluorophényl)-acryloyl]cinnamique et le chlorhydrate de fasudil.

2. Composition pharmaceutique pour son utilisation pour récupérer la sensibilité cornéenne, ladite composition comprenant un composé choisi parmi la 2-chloro-6,7-diméthoxy-N-[5-(1)-indazolyl]-quinazoline-4-amine, le dichlorhydrate de N-(1-benzyl-4-pipéridinyl)-1H-indazole-5-amine, l'acide 4-[2-(2,3,4,5,6-pentafluorophényl)acryloyl]cinnamique et le chlorhydrate de fasudil.

3. Composition pharmaceutique pour son utilisation pour traiter le syndrome de l'oeil sec, ladite composition comprenant un composé choisi parmi la 2-chloro-6,7-diméthoxy-N-[5-(1)-indazolyl]-quinazoline-4-amine, le dichlorhydrate de N-(1-benzyl-4-pipéridinyl)-1H-indazole-5-amine, l'acide 4-[2-(2,3,4,5,6-pentafluorophényl)acryloyl]cinnamique et le chlorhydrate de fasudil.

4. Utilisation d'un composé choisi parmi la 2-chloro-6,7-diméthoxy-N-[5-(1)-indazolyl]quinazoline-4-amine, le dichlorhydrate de N-(1-benzyl-4-pipéridinyl)-1H-indazole-5-amine, l'acide 4-[2-(2,3,4,5,6-pentafluorophényl)acryloyl]cinnamique et le chlorhydrate de fasudil pour la production d'une composition pharmaceutique destinée à favoriser la neurogenèse cornéenne.

5. Utilisation d'un composé choisi parmi la 2-chloro-6,7-diméthoxy-N-[5-(1)-indazolyl]quinazoline-4-amine, le dichlorhydrate de N-(1-benzyl-4-pipéridinyl)-1H-indazole-5-amine, l'acide 4-[2-(2,3,4,5,6-pentafluorophényl)acryloyl]cinnamique et le chlorhydrate de fasudil pour la production d'une composition pharmaceutique destinée à favoriser l'extension de l'axone du nerf cornéen.

6. Utilisation d'un composé choisi parmi la 2-chloro-6,7-diméthoxy-N-[5-(1)-indazolyl]quinazoline-4-amine, le dichlorhydrate de N-(1-benzyl-9-pipéridinyl)-1H-indazole-5-amine, l'acide 4-[2-(2,3,4,5,6-pentafluorophényl)acryloyl]cinnamique et le chlorhydrate de fasudil pour la production d'une composition pharmaceutique destinée à récupérer la sensibilité cornéenne.

7. Utilisation d'un composé choisi parmi la 2-chloro-6,7-diméthoxy-N-[5-(1)-indazolyl]quinazoline-4-amine, le dichlorhydrate de N-(1-benzyl-4-pipéridinyl)-1H-indazole-5-amine, l'acide 4-[2-(2,3,4,5,6-pentafluorophényl)acryloyl]cinnamique et le chlorhydrate de fasudil pour la production d'une composition pharmaceutique destinée à traiter le syndrome de l'oeil sec.
